# DEMANDE DE BREVET EUROPEEN

(11) **EP 4 643 837 A1**
(43) Date de publication de la demande: **05.11.2025**
(21) Numéro de dépôt: 25172770.7
(22) Date de dépôt: 26.04.2025
(51) Int. Cl.: A61G 13/12, A61H 15/02, A61H 99/00, A61N 5/06

(54) **EQUIPEMENT POUR UNE TABLE DE MASSAGE**

(30) Priorité: 29.04.2024 FR 2404437
(71) Demandeur: Lucibel SA, 76770 Le Houlme (FR)
(72) Inventeur: MOA, Yacine, 76510 Saint Nicolas d'Aliermont (FR); DAVID, Thibault, 76240 BONSECOURS (FR)
(74) Mandataire: Martin, Marie-Aude

(57) **Abrégé**

L'équipement (10) comprend un corps (12) délimitant une paroi proximale (14) pourvue d'une ouverture proximale (20P) destinée à être montée sur la table de massage sensiblement en coïncidence avec l'ouverture faciale (102) et une paroi distale (16) opposée pourvue d'une ouverture distale (20D), les deux parois (14, 16) étant reliées entre elles par une paroi périphérique (18) de façon à délimiter une cavité (22), la cavité comprenant une surface interne émettrice de lumière s'étendant autour de l'ouverture proximale (20P) pour émettre un faisceau de lumière primaire passant au travers de l'ouverture distale (20D) et une surface interne réflectrice de lumière agencée pour réfléchir un faisceau de lumière secondaire en direction de l'ouverture proximale (20P).

## Description

La présente invention concerne un équipement pour une table de massage dotée d'une ouverture faciale. Elle s'applique plus particulièrement mais non exclusivement au domaine des soins et massages corporels tels que pratiqués dans des salons d'esthétiques, de massage, dans les hammams, en thalassothérapie, en kinésithérapie, et plus généralement à toute pratique impliquant l'utilisation d'une table de massage.

Pour recevoir un massage ou un soin corporel, il est connu d'utiliser une table de massage formée principalement par un plateau horizontal qui permet à une personne de recevoir un massage en position couchée. Ce plateau est par exemple monté sur un pied de support pouvant être réglable avec une tête dont l'inclinaison est modifiable par rapport à un plan horizontal.

En général, le plateau est muni d'une ouverture pour recevoir le contour du visage de la personne installée sur la table. Cette solution est avantageuse car la personne installée sur la table de massage peut placer sa tête dans l'ouverture d'une manière confortable, avec son visage tourné vers le sol et soutenu par le bord de l'ouverture.

En outre, de façon optionnelle, pour plus de confort, le visage peut être traditionnellement protégé sur la table de massage par une housse matelassée et percée au niveau de l'ouverture et qui peut recouvrir l'intégralité de la table, ou encore également par une housse de têtière jetable, par un coussin en forme de U, par un pad visage troué, ou par une ou plusieurs serviettes roulées et disposées autour de l'ouverture.

Lorsque le soin ou le massage a une durée assez longue, il est prévu divers équipements permettant de mettre à profit le temps de soin ou de massage pour se divertir ou se relaxer par exemple en écoutant de la musique.

### Description de l'invention

A cet effet, l'invention a pour objet un équipement pour une table de massage pourvue d'une ouverture faciale, ledit équipement comprenant un corps délimitant une paroi proximale pourvue d'une ouverture proximale destinée à être montée sur la table de massage sensiblement en coïncidence avec l'ouverture faciale et une paroi distale opposée pourvue d'une ouverture distale, les deux parois étant reliées entre elles par une paroi périphérique de façon à délimiter une cavité, la cavité comprenant une surface interne émettrice de lumière s'étendant autour de l'ouverture proximale pour émettre un faisceau de lumière primaire selon un premier chemin optique passant au travers de l'ouverture distale et une surface interne réflectrice de lumière agencée pour réfléchir un faisceau de lumière secondaire selon un deuxième chemin optique en direction de l'ouverture proximale.

L'équipement selon l'invention permet de fournir un traitement par la lumière à l'utilisateur tout en garantissant un confort visuel pendant la séance de photothérapie. En effet, la projection de la lumière au sol simultanément à la projection de la lumière au niveau du visage de l'utilisateur permet de garantir une cohérence visuelle à l'utilisateur qui voit un éclairement au sol et en reçoit également sur son visage lors de sa séance.

Un dispositif selon l'invention peut comprendre l'une ou plusieurs des caractéristiques suivantes.

Dans un mode de réalisation préféré de l'invention, la paroi proximale forme un méplat sur le corps de l'équipement.

Dans un mode de réalisation préféré de l'invention, l'ouverture proximale est plus étroite que l'ouverture distale de telle sorte que la surface interne émettrice de lumière s'étend principalement à l'aplomb de l'ouverture distale.

Dans un mode de réalisation préféré de l'invention, la surface interne réflectrice de lumière est agencée autour de l'ouverture distale.

Dans un mode de réalisation préféré de l'invention, les deux ouvertures coïncident au moins partiellement selon un axe d'alignement de sorte que le corps présente un passage traversant ouvert selon cet axe.

Dans un mode de réalisation préféré de l'invention, la surface interne réflectrice est conformée selon une forme sensiblement d'ellipsoïde de révolution autour de l'ouverture distale.

Dans un mode de réalisation préféré de l'invention, la cavité a une forme générale d'ellipsoïde aplatie et oblate.

Dans un mode de réalisation préféré de l'invention, la surface émettrice et la surface réflectrice sont agencées de manière à ce qu'en position de montage sur la table, le rapport entre l'éclairement au niveau du sol par le premier faisceau de lumière et l'éclairement au niveau de l'ouverture proximale par le deuxième faisceau soit compris entre 0.5 et 1.2.

Dans un mode de réalisation préféré de l'invention, la source de lumière est accordable en longueur d'onde et fait partie d'un dispositif d'éclairage circadien configuré pour fournir une pluralité de scénarios d'éclairage de synchronisation prédéterminés d'une horloge biologique d'un individu.

Dans un mode de réalisation préféré de l'invention, l'une au moins des ouvertures distale et proximale a une forme générale oblongue.

Dans un mode de réalisation préféré de l'invention, le premier chemin optique est direct sans réflexion optique et le deuxième chemin optique est indirect avec au moins deux réflexions optiques.

L'invention a encore pour objet une table de massage comprenant un équipement selon l'invention, l'ouverture proximale est montée en vis-à-vis de l'ouverture faciale de la table et l'ouverture distale.

Le terme "spectre" doit être compris comme désignant une ou plusieurs fréquences (ou longueurs d'onde) de rayonnement produites par une ou plusieurs sources lumineuses. En conséquence, le terme "spectre" se réfère à des fréquences (ou longueurs d'onde) non seulement dans le domaine visible, mais aussi à des fréquences (ou longueurs d'onde) dans l'infrarouge, l'ultraviolet et d'autres domaines du spectre électromagnétique global. De même, un spectre donné peut avoir une largeur de bande relativement étroite ou une largeur de bande relativement large. Il convient également de noter qu'un spectre donné peut être le résultat d'un mélange de deux ou plusieurs autres spectres (par exemple, mélange de rayonnements émis respectivement par plusieurs sources lumineuses).

Dans le cadre de la présente divulgation, le terme "couleur" est utilisé de manière interchangeable avec le terme "spectre". Toutefois, le terme "couleur" est généralement utilisé pour faire référence à une propriété du rayonnement qui est perceptible par un observateur (bien que cette utilisation ne soit pas destinée à limiter la portée de ce terme). Il convient également de noter que le terme "couleur" peut être utilisé en relation avec la lumière blanche et la lumière non blanche.

Le terme "température de couleur" se réfère essentiellement à une teneur en couleur ou à une nuance particulière (par exemple, rougeâtre, bleuâtre) de la lumière blanche. La température de couleur d'un échantillon de rayonnement donné est généralement caractérisée par la température en degrés Kelvin (K) d'un radiateur à corps noir qui émet essentiellement le même spectre que l'échantillon de rayonnement en question. Les températures de couleur inférieures indiquent généralement une lumière blanche ayant une composante rouge plus importante ou une "sensation de chaleur", tandis que les températures de couleur supérieures indiquent généralement une lumière blanche ayant une composante bleue plus importante ou une "sensation de fraîcheur". À titre d'exemple, le feu a une température de couleur d'environ 1800 degrés Kelvin, une ampoule à incandescence classique a une température de couleur d'environ 2848 degrés Kelvin, la lumière du jour tôt le matin a une température de couleur d'environ 3000 degrés Kelvin, et le ciel couvert de midi a une température de couleur d'environ 10000 degrés Kelvin.

Les termes "dispositif d'éclairage" ou "luminaire" sont utilisés ici de manière interchangeable pour désigner une mise en œuvre ou un arrangement d'une ou plusieurs unités d'émission de lumière dans un facteur de forme, un assemblage ou un emballage particulier. Le terme "unité d'émission de lumière" est utilisé ici pour désigner un appareil comprenant une ou plusieurs sources lumineuses de types identiques ou différents. Une unité d'émission de lumière donnée peut avoir une variété de dispositions de montage pour la ou les sources lumineuses, de dispositions et de formes de boîtiers et/ou de configurations de connexions électriques et mécaniques. En outre, une unité d'émission de lumière donnée peut éventuellement être associée à (par exemple, inclure, être couplée à et/ou emballée avec) divers autres composants (par exemple, circuit de contrôle) relatifs au fonctionnement de la ou des sources lumineuses. Une "unité d'émission de lumière à base de LED" est une unité d'émission de lumière qui comprend une ou plusieurs sources lumineuses à base de LED telles que décrites ci-dessus, seules ou en combinaison avec d'autres sources lumineuses non basées sur des LED. Une unité d'éclairage "multicanal" désigne une unité d'émission de lumière à base de LED ou non à base de LED qui comprend au moins deux sources lumineuses configurées pour générer respectivement différents spectres de rayonnement, chaque spectre de source différent pouvant être désigné comme un "sous-canal" de l'unité d'éclairage multicanal.

Le terme "contrôleur" est utilisé ici de manière générale pour décrire divers appareils relatifs au fonctionnement d'une ou de plusieurs sources lumineuses. Un contrôleur peut être mis en œuvre de nombreuses façons (par exemple, avec du matériel spécialisé) pour exécuter les diverses fonctions décrites dans le présent document. Un "processeur" est un exemple de contrôleur qui utilise un ou plusieurs microprocesseurs pouvant être programmés à l'aide d'un logiciel (par exemple, un microcode) pour exécuter les diverses fonctions décrites dans le présent document. Un contrôleur peut être mis en œuvre avec ou sans processeur, et peut également être mis en œuvre comme une combinaison de matériel dédié pour exécuter certaines fonctions et un processeur (par exemple, un ou plusieurs microprocesseurs programmés et des circuits associés) pour exécuter d'autres fonctions. Les exemples de composants de contrôleur qui peuvent être employés dans divers modes de réalisation de la présente divulgation comprennent, sans s'y limiter, des microprocesseurs conventionnels, des circuits intégrés à application spécifique (ASIC).

Dans diverses mises en œuvre, un processeur ou un contrôleur peut être associé à un ou plusieurs supports de stockage (génériquement appelés ici "mémoire", par exemple, mémoire informatique volatile et non volatile telle que RAM, PROM, EPROM et EEPROM, disquettes, disques compacts, disques optiques, bandes magnétiques, etc.) Dans certaines réalisations, le support de stockage peut être codé avec un ou plusieurs programmes qui, lorsqu'ils sont exécutés par un ou plusieurs processeurs et/ou contrôleurs, exécutent au moins certaines des fonctions décrites dans le présent document. Divers supports de stockage peuvent être fixés dans un processeur ou un contrôleur ou peuvent être transportables, de sorte que le ou les programmes qui y sont stockés peuvent être chargés dans un processeur ou un contrôleur afin de mettre en œuvre divers aspects de la présente invention.

Les termes logiciel, programme ou programme informatique sont utilisés ici dans un sens générique pour désigner tout type de code informatique (par exemple, logiciel ou microcode) qui peut être utilisé pour programmer un ou plusieurs processeurs ou contrôleurs.

Le terme "interface utilisateur" tel qu'il est utilisé ici fait référence à une interface entre un utilisateur ou un opérateur humain et un ou plusieurs dispositifs qui permet la communication entre l'utilisateur et le(s) dispositif(s). Les exemples d'interfaces utilisateur qui peuvent être utilisées dans diverses mises en œuvre de la présente divulgation comprennent, sans s'y limiter, des commutateurs, des potentiomètres, des boutons, des cadrans, des curseurs, une souris, un clavier, un pavé numérique, divers types de contrôleurs de jeu (par exemple, des manettes), des billes de piste, des écrans d'affichage, divers types d'interfaces utilisateur graphiques, des écrans tactiles, des microphones et d'autres types de capteurs qui peuvent recevoir une certaine forme de stimulus généré par l'homme et générer un signal en réponse à ce stimulus.

Tel qu'il est utilisé dans le présent document, le terme « DEL » ou « LED » doit être compris comme incluant toute diode électroluminescente ou tout autre type de système basé sur l'injection/jonction de porteurs capable de générer un rayonnement en réponse à un signal électrique. Ainsi, le terme LED comprend, sans s'y limiter, diverses structures à base de semi-conducteurs qui émettent de la lumière en réponse à un courant, des polymères émetteurs de lumière, des diodes électroluminescen tes organiques (OLED), des bandes électroluminescentes, etc. En particulier, le terme LED fait référence aux diodes électroluminescentes de tous types (y compris les diodes électroluminescentes semi-conductrices et organiques) qui peuvent être configurées pour générer un ou plusieurs rayonnements dans le spectre infrarouge, le spectre ultraviolet et diverses parties du spectre visible (comprenant généralement des longueurs d'onde de rayonnement allant d'environ 400 nanomètres à environ 700 nanomètres). Parmi les exemples de diodes électroluminescentes, on peut citer, sans s'y limiter, divers types de diodes infrarouges, de diodes ultraviolettes, de diodes rouges, de diodes bleues, de diodes vertes, de diodes jaunes, de diodes ambres, de diodes orange et de diodes blanches. Il convient également de noter que les LED peuvent être configurées et/ou contrôlées pour générer un rayonnement ayant différentes largeurs de bande (par exemple, largeur complète à mi-maximum, ou FWHM) pour un spectre donné (par exemple, largeur de bande étroite, largeur de bande large) et une variété de longueurs d'onde dominantes au sein d'une catégorisation de couleur générale donnée.

Le terme "source lumineuse" doit être compris comme se référant à une ou plusieurs sources de rayonnement, y compris, mais sans s'y limiter, les sources à base de LED (y compris une ou plusieurs LED telles que définies ci-dessus), les sources incandescentes (par exemple, les lampes à filament, les lampes halogènes), les lampes à incandescence, les lampes à incandescence, etc, lampes à filament, lampes halogènes), sources fluorescentes, sources phosphorescentes, sources à décharge à haute intensité (par exemple, lampes à vapeur de sodium, à vapeur de mercure et à halogénures métalliques), lasers, autres types de sources électroluminescentes, sources pyro-luminescentes (par exemple, flammes), lampes à bougies, etc, flammes), sources luminescentes de bougies (par exemple, manchons de gaz, sources de rayonnement d'arc de carbone), sources photo-luminescentes (par exemple, sources de décharge gazeuse), sources luminescentes de cathode utilisant la satiation électronique, sources galvano-luminescentes, sources cristallo-luminescentes, sources ciné-luminescentes, sources thermo-luminescentes, sources triboluminescentes, sources sonoluminescentes, sources radioluminescentes, et polymères luminescents.

Une source lumineuse donnée peut être configurée pour générer un rayonnement électromagnétique dans le spectre visible, en dehors du spectre visible, ou une combinaison des deux.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig.1] La [Fig.1] représente une vue en coupe longitudinale d'une table de massage comprenant un équipement selon l'invention.
[Fig.2] La [Fig.2] représente une vue de dessous et en perspective de l'équipement de la [Fig.1].
[Fig.3] La [Fig.3] représente une vue de dessous de l'équipement de la [Fig.1].
[Fig.4] La [Fig.4] représente une vue de dessus de l'équipement de la [Fig.1].
[Fig.5] La [Fig.5] représente une vue de côté de l'équipement de la [Fig.1].
[Fig.6] La [Fig.6] représente une vue en coupe transversale de la table de massage et de l'équipement de l'invention.
[Fig.7] La [Fig.7] représente une vue en écorché de l'équipement de l'invention agencé dans la table de massage.
[Fig.8] La [Fig.8] représente une vue schématique d'un dispositif d'éclairage circadien.

### Description détaillée de l'invention

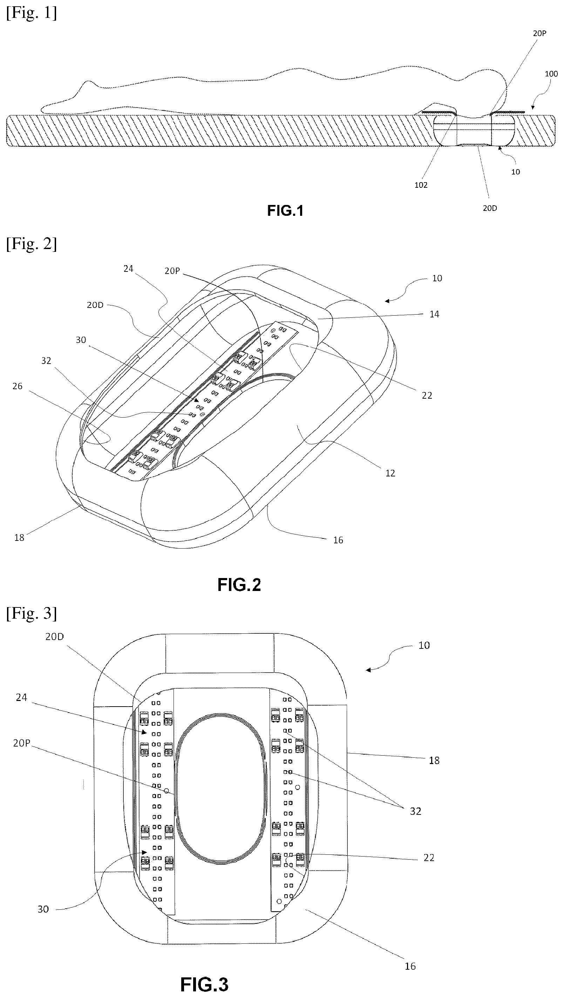

On a représenté sur la [Fig.1] une table de massage selon l'invention dans laquelle est agencé un équipement. Dans la suite de la description, la table de massage sera désignée par la référence générale 100 et l'équipement par la référence générale 10.

L'équipement 10 est particulièrement destiné aux salons d'esthétiques, de massage, aux hammams, à la thalassothérapie, à la kinésithérapie, en somme à toute pratique impliquant une utilisation d'une table de massage.

Comme cela est illustré sur la [Fig.1], la table de massage 100 comprend une ouverture faciale 102. Par exemple, lorsqu'une personne est allongée sur la table de massage 100, son visage repose sur des rebords périphériques de l'ouverture faciale 102. La table de massage 100 peut comprendre une structure de support classique comprenant par exemple un pied réglable supportant un plateau horizontal sur lequel est articulé un dossier de tête articulé (non représenté sur les figures).

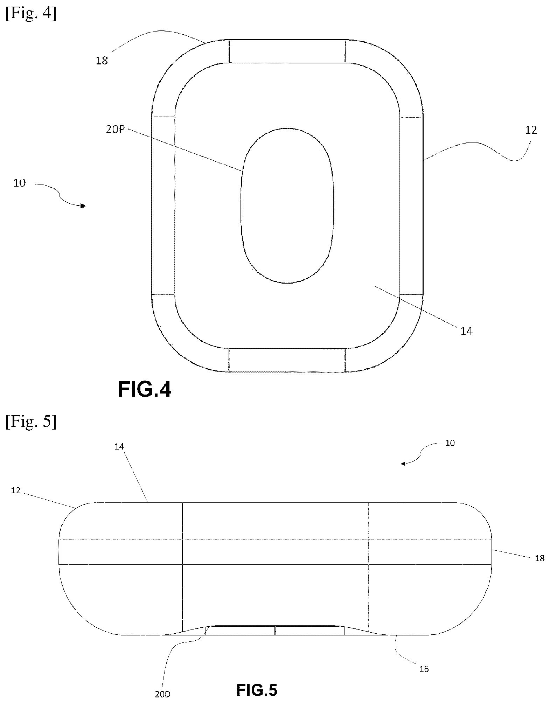

Comme cela est illustré sur les figures 2 et 5, l'équipement 10 comprend un corps 12 délimitant une paroi proximale 14 pourvue d'une ouverture proximale 20P destinée à être montée sur la table de massage 100, de préférence sensiblement en coïncidence avec l'ouverture faciale 102.

L'équipement 10 comprend encore une paroi distale 16 opposée pourvue d'une ouverture distale 20D. Dans l'exemple décrit, les deux parois proximale 14 et distale 16 sont reliées entre elles par une paroi périphérique 18 de façon à délimiter une cavité 22.

Comme cela ressort des figures, en particulier des figures 2 et 5, la cavité 22 a de préférence une forme générale d'ellipsoïde aplatie et oblate. De préférence, la paroi proximale 20P forme en outre un méplat comme cela est visible sur la [Fig.5]. En outre, de préférence, chaque ouverture proximale 20P ou distale 20D a une forme générale oblongue. L'ouverture proximale 20P a par exemple une forme ovoïde selon la forme ovale d'un visage humain ([Fig.4]).

Selon l'invention, la cavité 22 comprend une surface interne émettrice 24 de lumière formant une source de lumière 30 de l'équipement 10. Cette surface interne émettrice de lumière 24 s'étend de préférence au moins partiellement autour de l'ouverture proximale 20P pour émettre un faisceau de lumière primaire selon un premier chemin optique c1 passant au travers de l'ouverture distale 20D.

En outre, la cavité 22 comprend dans l'exemple décrit une surface interne réflectrice 26 de lumière agencée pour réfléchir un faisceau de lumière secondaire provenant de la source de lumière 30 selon un deuxième chemin optique c2 en direction de l'ouverture proximale 20P.

La surface interne réflectrice de lumière 26 est agencée de préférence au moins partiellement autour de l'ouverture distale 20D. La surface interne réflectrice 26 est par exemple conformée selon une forme sensiblement d'ellipsoïde de révolution autour de l'ouverture distale 20D en suivant la courbure intérieure de la cavité 22. De préférence, la cavité 22 peut être revêtue intégralement ou partiellement par un revêtement réfléchissant ou être formée dans un matériau réfléchissant les longueurs d'onde du spectre de lumière de la source 30.

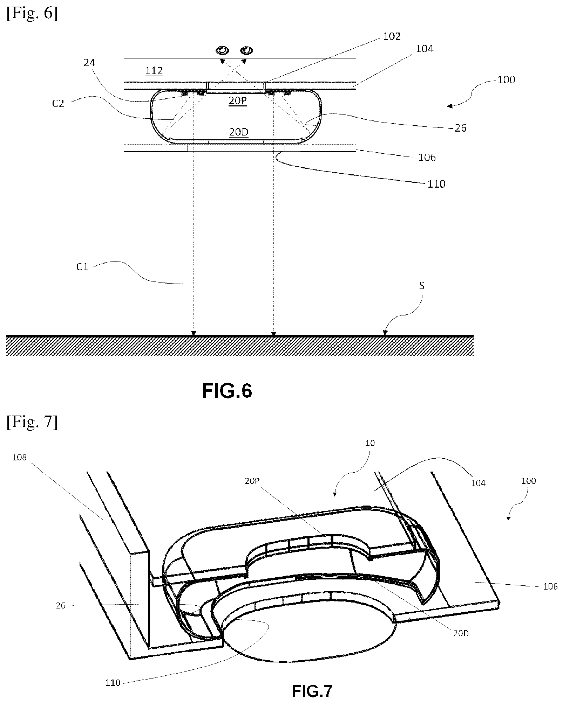

De préférence, le premier chemin optique c1 est direct sans réflexion optique jusqu'au sol S et le deuxième chemin optique c2 est indirect avec au moins deux réflexions optiques jusqu'au visage de l'utilisateur par l'ouverture proximale 20P ([Fig.6]).

Dans l'exemple décrit, la surface interne émettrice 24 forme la source de lumière 30 qui est générée par une pluralité d'unités 28 d'émission de lumière qui vont être décrites ci-après de façon plus détaillée. Dans le mode de réalisation préféré de l'invention, chaque unité 28 comprend au moins un élément lumineux 32, par exemple une diode électroluminescente (DEL ou selon l'acronyme anglais LED), et de préférence comprend une pluralité d'éléments lumineux pour chaque unité 28.

De préférence, les deux ouvertures 20P, 20D coïncident au moins partiellement selon un axe d'alignement de sorte que le corps 12 présente un passage traversant ouvert selon cet axe. La personne qui positionne son visage sur l'ouverture proximale 20P peut ainsi voir le sol S au travers du corps 12 de l'équipement 10.

De préférence, l'ouverture proximale 20P est plus étroite que l'ouverture distale 20D de telle sorte que la surface interne émettrice de lumière 24 s'étend principalement à l'aplomb de l'ouverture distale 20D. Ceci permet à la source de lumière 30 de projeter un faisceau de lumière direct selon le chemin optique c1 sur le sol S. Par exemple, la surface émettrice de lumière 24 se présente comme cela est illustré sur la [Fig.3] sous forme d'au moins une bande de plaque de circuit imprimé sur laquelle sont montées les éléments lumineux 32.

La surface émettrice 24 et la surface réflectrice 26 sont configurées de manière à ce qu'en position de montage sur la table 100, l'éclairement au niveau du sol par le faisceau de lumière primaire (selon le chemin optique c1) soit sensiblement égal à l'éclairement au niveau de l'ouverture proximale 20P produit principalement par le faisceau de lumière secondaire (selon le chemin optique c2).

On entendra dans l'exemple décrit, par l'expression sensiblement égal, que le rapport entre l'éclairement au sol S et l'éclairement à l'ouverture proximale 20P est compris entre 0.5 et 1.2 et de préférence entre 0.7 et 1. Par exemple, l'éclairement au sol est d'environ 1000 lux et l'éclairement à l'ouverture proximale 20P est d'environ 1400 lux. Dans ce cas, le rapport est de 0.7. Ceci permet de garantir une cohérence visuelle pour l'utilisateur qui reçoit ainsi sur son visage, à l'ouverture proximale 20P, un éclairement presque équivalent à celui qu'il perçoit au niveau du sol S. Cela apporte un confort visuel à l'utilisateur. La table de massage 100 est par exemple située entre 50 à 70 centimètres au-dessus du sol S.

En outre, dans un mode de réalisation préféré illustré sur la [Fig.6], l'équipement 10 s'étend à l'intérieur de la table de massage 100. Par exemple, la table de massage 100 comprend des parois supérieure 104, inférieure 106 et latérale 108 pour former un coffre. La paroi inférieure 106 comprend par exemple une ouverture 110 et la paroi supérieure 104 comprend par exemple l'ouverture 102. De préférence, comme illustré sur les figures 6 et 7, l'équipement 10 est intégré à l'intérieur du coffre de telle sorte que l'ouverture proximale 20P coïncide avec l'ouverture 102 et l'ouverture distale 20D coïncide avec l'ouverture 110. En outre, par exemple, la table de massage 100 comprend une housse matelassée 112.

Dans un mode de réalisation préféré de l'invention, de préférence, l'équipement 10 produit un éclairage à fonction d'éclairage circadien efficace pour synchroniser l'horloge biologique d'un individu. Bien entendu, en variante, l'équipement 10 peut produire un éclairage avec d'autres fonctions de traitement par la lumière, par exemple une fonction de photothérapie ou de luminothérapie.

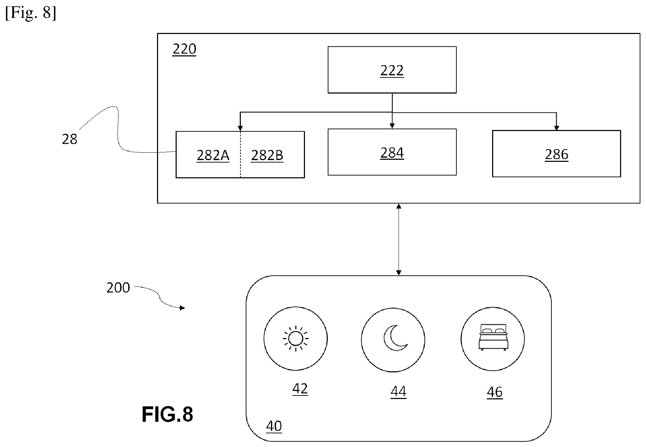

Par exemple, la source de lumière 30 peut être accordable et faire partie d'un dispositif 200 d'éclairage circadien ([Fig.8]) configuré pour fournir une pluralité de scénarios d'éclairage de synchronisation d'une horloge biologique d'un individu. En particulier, le dispositif 200 est configuré pour modifier la température de couleur de la lumière en fonction du temps.

Les scénarios d'éclairage peuvent consister de façon plus particulière mais non exclusive en une synchronisation d'une horloge biologique (luminothérapie), une stimulation du corps en vue d'une épreuve sportive ou intellectuelle, un décalage de l'horloge biologique en prévision d'un voyage futur ou une récupération d'un décalage horaire lié à un voyage récent, un protocole d'optimisation de la concentration, de la performance intellectuelle ou physique et des capacités neurocognitives, ou en une aide à l'endormissement.

La [Fig.8] représente de façon schématique le dispositif 200. En particulier, le dispositif 200 comprend une première unité 282 d'émission de lumière de couleur blanche. Cette première unité 282 émet une lumière dont le spectre lumineux s'étend principalement dans une plage de longueurs d'onde comprise entre 400 nm et 800 nm.

Selon l'invention, la première unité 282 comprend une pluralité des éléments lumineux 32 émettant chacun un flux lumineux unitaire en direction d'une surface à éclairer. De préférence, la première unité 282 est formée par une combinaison d'au moins deux groupes 282A, 282B ou sous-canaux d'éléments lumineux, ici des diodes électroluminescentes : un premier groupe ou sous-canal 282A de diodes électroluminescentes émettant une lumière de type blanc chaud avec une température de couleur d'environ 2700K et un deuxième groupe ou sous-canal 212B de diodes électroluminescentes émettant une lumière de type blanc froid avec une température de couleur d'environ 6500K. Ces sous-canaux 282A et 282B peuvent être pilotés de façon indépendante par un module de commande 220.

En outre, de préférence, le dispositif 200 comprend une deuxième unité d'émission de lumière 214 de couleur bleue dont le spectre lumineux forme un pic étroit et dont le sommet du pic est compris entre 400 et 500 nm formant une deuxième unité dit de stimulation. Ce pic est de préférence centré autour de 490 nm, avec une marge de plus ou moins 5 nm autour de cette valeur. De préférence, par pic étroit, on entend que la largeur à mi-hauteur du pic étroit est comprise entre 20 et 50 nm. La valeur préférée de 490 nanomètres correspond à un maximum de sensibilité des cellules réceptrices non-visuelles ou glandes à mélanopsine d'un être humain.

Plus spécifiquement, la puissance lumineuse émise par la deuxième unité 214 est toujours inférieure ou égale à 35% de la puissance totale émise par la source lumineuse 30 du dispositif 200. Ceci permet d'optimiser le rendu des couleurs du dispositif 200 sans dénaturer la couleur blanche de la lumière émise par la source 30 tout en permettant une action efficace sur les cellules réceptrices non-visuelles.

En outre, de préférence, le dispositif 200 comprend encore une troisième unité 286 dont le spectre lumineux est relativement enrichi de longueurs d'onde supérieures à 560 nm et relativement dépourvu de longueurs d'onde inférieures à 560 nm. De préférence, le spectre lumineux de la troisième unité 286 est formé par un pic centré sur une longueur d'onde supérieure à 560 nm, de préférence centrée sur 590 nm (avec une marge de plus ou moins 5 nm autour de cette valeur), avec une largeur de pic comprise entre 20 et 50 nm. Cette unité 286 permet d'activer le système visuel mais n'a pas d'effet sur les cellules réceptrices non visuelles à mélanopsine d'un être humain. La lumière ambre émise par cette troisième unité 286 permet au corps humain de sécréter naturellement la mélatonine par inhibition des cellules réceptrices à mélanopsine.

De préférence, afin de tenir compte du rythme ultradien de l'être humain, la durée d'un scénario est comprise entre 80 minutes et 100 minutes, de préférence 90 minutes.

Par exemple, le module de commande 220 comprend des moyens de génération des instructions logicielles de pilotage de la source de lumière à partir d'une trame de données de résultats d'un ensemble de données d'évaluation d'un profil d'un utilisateur.

En outre, de préférence, le dispositif 200 comprend de préférence un terminal de commande à distance du module 220 de commande du dispositif 200. De préférence, le dispositif 200 comprend une interface utilisateur 400, supportée par exemple de manière logicielle par le terminal de commande à distance.

Le module de commande 220 comprend des moyens de génération des signaux de pilotage des unités d'éclairage 28 en fonction d'une trame de données générée à la suite de la sélection par un opérateur d'un scénario parmi une pluralité de scénarios prédéterminés. Ces instructions logicielles permettent par exemple la génération de signaux de pilotage adressés à l'unité d'adressage puis aux unités 210. L'interface utilisateur 40 est par exemple supportée de manière logicielle sur l'équipement électronique externe qui communique avec le dispositif 200. Le module de commande 220 comprend un processeur pour exécuter des instructions logicielles pour la génération de signaux de pilotage des unités 28. En variante, l'interface utilisateur 40 peut être intégrée dans un même boîtier d'emballage du dispositif 200 sans terminal de commande fonctionnant à distance.

Par exemple, les instructions logicielles sont générées à partir de l'interface utilisateur 40 du dispositif 200 permettant à un opérateur de sélectionner les paramètres de réglage de la puissance de chaque unité 28 en fonction d'un profil de l'utilisateur. De préférence, l'interface utilisateur 40 est supportée de manière logicielle sur une tablette. La tablette communique par exemple avec le module de commande 220 par une liaison filaire ou sans fil (par exemple par Bluetooth^{®} ou par WiFi).

Dans un mode de réalisation préféré de l'invention, le dispositif 200 comprend un module de recommandation configuré pour générer un niveau de recommandation pour chaque scénario parmi la pluralité de scénarios prédéterminés avec un créneau horaire d'application de chacun des scénarios.

De préférence, ledit module de recommandation comprend un moyen de saisie pour recueillir des données relatives à un utilisateur et un moyen de détermination d'un scénario à partir d'une combinaison des données saisies et d'une information d'un créneau horaire local actuel. Les moyens de détermination comprennent par exemple une table de correspondance prenant en entrée d'une part les données utilisateurs et les créneaux horaires d'une journée et fournissant en sortie des niveaux de recommandation pour les trois scénarios (ou plus).

Les données relatives à l'utilisateur comprennent par exemple une liste de paramètres choisis parmi un chronotype (matinal, vespéral ou mixte), une heure de réveil et/ou une heure de coucher, un décalage horaire lié à la traversée de plusieurs fuseaux horaires vers l'Est ou vers l'Ouest et un déficit de sommeil.

Dans un mode de réalisation préféré de l'invention, l'interface utilisateur 40 comprend une pluralité d'icônes représentant chacun un scénario. Dans l'exemple illustré sur la [Fig.1], l'interface utilisateur 40 comprend des première 42, deuxième 44 et troisième 46 icônes correspondant respectivement à un scénario de réveil, un scénario de relaxation dynamique et un scénario de récupération qui vont être détaillés ci-après.

Le module de recommandation comprend par exemple des moyens visuels d'affichage différencié des icônes entre elles, par exemple une mise en surbrillance ou un affichage d'un score de recommandation sur une échelle de 0% à 100% associé à chaque icône.

Dans un mode de réalisation préféré de l'invention, dans un scénario de stimulation, le module de commande 220 est configuré pour faire varier progressivement la puissance lumineuse de la deuxième unité d'une puissance nulle jusqu'à une puissance maximale correspondant à au plus 35% de la puissance totale de la source lumineuse.

On va également décrire trois scénarios de synchronisation d'une horloge biologique d'un utilisateur. De préférence, quel que soit le scénario, initialement, la source de lumière 30 est accordée spectralement de préférence à une lumière blanche chaud d'accueil : par exemple, éclairement de 100 lux et température de couleur blanc chaud de 2700K.

Dans un premier scénario de réveil, au cours d'une première période T1, les unités 282A et 286 respectivement de lumière blanc chaud et de lumière ambre sont activées de telle sorte que la température de couleur décroît jusqu' à atteindre une température de couleur de 2100K. Pendant cette période T1, la lumière émise par la source 200 tend à reproduire le lever du soleil ou l'aube ce qui permet à l'individu de synchroniser son horloge biologique sur un début de matinée. Puis, au cours d'une période T2, l'unité de lumière ambre 286 est désactivée et le sous-canal de l'unité de lumière blanche 212B de 6500K est activée de sorte que la température de couleur de la source 30 croît d'une température de 2700K à 6500K.

En outre, la puissance de l'unité de lumière cyan 284 est progressivement augmentée d'une valeur sensiblement nulle jusqu'à atteindre une valeur maximale inférieure à 35% de la puissance totale de la source de lumière 30 dans une période T3. De préférence, le module de commande 220 est configuré pour faire varier progressivement la puissance lumineuse de la deuxième unité 284 d'une puissance nulle jusqu'à une puissance maximale correspondant à au plus 35% de la puissance totale.

Dans un scénario de relaxation dynamique, le module de commande 220 est configuré pour faire varier la puissance lumineuse de la deuxième unité 284 entre une valeur nulle et une valeur maximale de puissance selon un profil de trains d'impulsions. Par exemple, pour une durée de traitement de 90 minutes, le profil de trains d'impulsions comprend entre quatre et huit impulsions, ici cinq impulsions. Les impulsions oscillent entre une température de couleur d'environ 2100 Kelvins (activation de l'unité ambre 286 et désactivation de l'unité de la lumière cyan 284) et une température de couleur d'environ 6000 Kelvins (activation de l'unité de la lumière cyan 284 et désactivation de la lumière de l'unité ambre 286). L'unité de lumière blanche 282 est de préférence maintenue activée au cours de ce scénario. En particulier, la valeur de puissance maximale de la lumière émise par l'unité 284 est toujours inférieure à 35% de la puissance totale de la source lumineuse 30. De préférence, la valeur de la puissance maximale de la lumière 284 correspond à 30% de la puissance totale de la source 30.

Enfin, dans un troisième scénario dit de récupération, de préférence, le module de commande 220 est configuré pour faire varier la puissance lumineuse de la première unité 282 jusqu'à une puissance sensiblement nulle pendant une période T1 et pour maintenir uniquement la puissance lumineuse de la troisième unité 286 à une valeur non nulle pendant une période T2. Pendant une période T3, les trois unités 282 à 286 sont progressivement activées pour augmenter progressivement la température de couleur de 1800K à 4000K. L'utilisation d'une lumière de plus en plus ambrée pendant la période T2 permet d'accompagner le corps à la sécrétion de mélatonine, ce qui va favoriser la relaxation.

On va maintenant décrire les principaux aspects de fonctionnement de l'équipement 10, en référence aux figures 1 à 8, par exemple en relation avec un utilisateur souhaitant bénéficier d'une séance de luminothérapie en rapport à avec son activité passée ou future.

Dans un premier temps, un opérateur procède à une récupération des données de l'utilisateur : chronotype (matinal, vespéral ou mixte), temps moyen de sommeil, déficit de sommeil et/ou événement passé ou à venir de décalage horaire. Le chronotype de l'utilisateur pourra être déterminé par exemple selon l'heure privilégiée de début de sommeil et l'heure privilégiée de réveil de l'utilisateur. Concernant l'événement de décalage horaire, l'opérateur indique par exemple un nombre de fuseaux horaires de décalage vers l'Est ou vers l'Ouest traversés par l'utilisateur. L'opérateur saisit ces données via par exemple l'interface utilisateur 40.

L'opérateur indique également par l'intermédiaire de l'interface utilisateur 40 le créneau horaire actuel local (par exemple 9h-11h, 11h-13h, 13h-15h, 15h-17h, ou 17h-19h). Ce créneau horaire local actuel peut également être calculé automatiquement en récupérant une heure actuelle locale d'une horloge interne du dispositif 20. Le module de recommandation calcule alors pour chaque scénario un score de recommandation qui s'affiche sur l'interface utilisateur 40 avec un créneau horaire recommandé pour chaque scénario. L'unité de couleur cyan 284 du dispositif 20 est activée dans chaque scénario d'éclairage et permet d'accentuer artificiellement la stimulation des voies non visuelles du corps humain ce qui impacte directement la sécrétion d'hormones.

L'utilisateur s'allonge sur la table 100 et positionne son visage à l'intérieur de l'ouverture proximale 20P.

La source de lumière 30 est ensuite activée selon le scénario recommandé pour l'utilisateur et produit un premier faisceau lumineux en direction du sol S et un deuxième faisceau en direction du visage de l'utilisateur à l'ouverture proximale 20P après une ou plusieurs réflexions à l'intérieur de la cavité 22.

Comme le rapport entre l'éclairement au niveau du sol S est l'éclairement à l'ouverture proximale 20P est compris entre de préférence 0.7 et 1, le confort visuel de l'utilisateur est garanti. Il y a en effet une cohérence visuelle entre l'éclairement perçu au sol et l'éclairement qu'il reçoit au niveau de ses yeux.

La séance peut être ainsi mise à profit pour régler l'horloge biologique de l'utilisateur grâce à l'équipement 10.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Equipement (10) pour une table (100) de massage pourvue d'une ouverture faciale (102), ledit équipement (10) comprenant un corps (12) délimitant une paroi proximale (14) pourvue d'une ouverture proximale (20P) destinée à être montée sur la table de massage sensiblement en coïncidence avec l'ouverture faciale (102) et une paroi distale (16) opposée pourvue d'une ouverture distale (20D), les deux parois étant reliées entre elles par une paroi périphérique (18) de façon à délimiter une cavité (22), la cavité (22) comprenant une surface interne émettrice de lumière (24) s'étendant autour de l'ouverture proximale (20P) pour émettre un faisceau de lumière primaire selon un premier chemin optique (c1) passant au travers de l'ouverture distale (20D) et une surface interne réflectrice (26) de lumière agencée pour réfléchir un faisceau de lumière secondaire selon un deuxième chemin optique (c2) en direction de l'ouverture proximale (20P).

2. Equipement (10) selon la revendication précédente, dans lequel la paroi proximale (14) forme un méplat sur le corps (12) de l'équipement (10).

3. Equipement (10) selon la revendication précédente, dans lequel l'ouverture proximale (20P) est plus étroite que l'ouverture distale (20D) de telle sorte que la surface interne émettrice de lumière (24) s'étend principalement à l'aplomb de l'ouverture distale (20D).

4. Equipement (10) selon l'une quelconque des revendications précédentes, dans lequel la surface interne réflectrice de lumière (26) est agencée autour de l'ouverture distale (20D).

5. Equipement (10) selon l'une quelconque des revendications précédentes, dans lequel les deux ouvertures (20P, 20D) coïncident au moins partiellement selon un axe d'alignement de sorte que le corps (12) présente un passage traversant ouvert selon cet axe.

6. Equipement (10) selon l'une quelconque des revendications précédentes, dans lequel la surface interne réflectrice (26) est conformée selon une forme sensiblement d'ellipsoïde de révolution autour de l'ouverture distale (20D).

7. Equipement (10) selon l'une quelconque des revendications précédentes, dans lequel la cavité (22) a une forme générale d'ellipsoïde aplatie et oblate.

8. Equipement (10) selon l'une quelconque des revendications précédentes, dans lequel la surface émettrice (24) et la surface réflectrice (26) sont agencées de manière à ce qu'en position de montage sur la table (100), le rapport entre l'éclairement au niveau du sol (S) par le premier faisceau de lumière et l'éclairement au niveau de l'ouverture proximale (20P) par le deuxième faisceau de lumière soit compris entre 0.5 et 1.2.

9. Equipement (10) selon l'une quelconque des revendications précédentes, dans lequel la source de lumière (30) est accordable en longueur d'onde et fait partie d'un dispositif (200) d'éclairage circadien configuré pour fournir une pluralité de scénarios d'éclairage de synchronisation prédéterminés d'une horloge biologique d'un individu.

10. Equipement (10) selon l'une quelconque des revendications précédentes, dans lequel l'une au moins des ouvertures distale (20D) et proximale (20P) a une forme générale oblongue.

11. Equipement (10) selon l'une quelconque des revendications précédentes, dans lequel le premier chemin optique (c1) est direct sans réflexion optique et le deuxième chemin optique (c2) est indirect avec au moins deux réflexions optiques.

12. Table (100) de massage comprenant un équipement selon l'une quelconque des revendications précédentes, l'ouverture proximale (20P) est montée en vis-à-vis de l'ouverture faciale (102) de la table (100) et l'ouverture distale (20D).
